(19) Europäisches Patentamt
European Patent Office
Office européen des brevets

(11) Veröffentlichungsnummer: **0 205 024**
**A2**

(12) # EUROPÄISCHE PATENTANMELDUNG

(21) Anmeldenummer: 86107124.9

(22) Anmeldetag: 26.05.86

(51) Int. Cl.[4]: C 07 D 401/14
C 07 D 417/14, C 07 D 413/14
A 01 N 43/653, A 01 N 43/82

(30) Priorität: 07.06.85 DE 3520328

(43) Veröffentlichungstag der Anmeldung:
17.12.86 Patentblatt 86/51

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Stetter, Jörg, Dr.
Gellertweg 4
D-5600 Wuppertal 1(DE)

(72) Erfinder: Schallner, Otto, Dr.
Noldeweg 22
D-4019 Monheim(DE)

(72) Erfinder: Gehring, Reinhold, Dr.
Dasnöckel 49
D-5600 Wuppertal 11(DE)

(72) Erfinder: Lindig, Markus, Dr.
Dahlienweg 16
D-4010 Hilden(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch-Gladbach 2(DE)

(54) **5-Amino-4-heterocyclyl-1-pyridyl-pyrazole.**

(57) Die Erfindung betrifft neue 5-Amino-4-heterocyclyl-1-pyridyl-pyrazole der Formel (I)

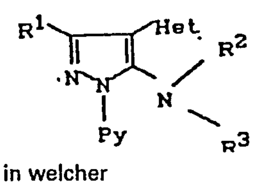

(I)

in welcher

$R^1$ für Wasserstoff oder Alkyl steht,

$R^2$ für Wasserstoff oder für einen Rest

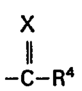 steht,

$R^3$ für Wasserstoff, für einen Rest

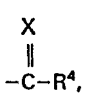, für

Alkyl, Alkenyl oder Alkinyl steht,

wobei

$R^4$ für Wasserstoff, Alkyl, Alkoxy, Alkylthio, Alkoxyalkyl, Alkylthioalkyl, Halogenalkyl, Alkenyl, Alkinyl, für Alkylamino für Dialkylamino, für gegebenenfalls substituiertes Cycloalkyl, für jeweils gegebenenfalls substituiertes Aryl, Aryloxy, Arylthio, Arylamino oder Aryloxyalkyl steht und

X für Sauerstoff oder Schwefel steht,

Py für gegebenenfalls substituiertes Pyridyl steht und

Het für einen gegebenenfalls substituierten gesätigen oder ungesättigten Heterocyclus steht,

mehrere Verfahren zu ihrer Herstellung und ihre Verwendung als Herbizide.

EP 0 205 024 A2

BAYER AKTIENGESELLSCHAFT 5090 Leverkusen, Bayerwerk
Konzernverwaltung RP
Patentabteilung KM/ABc
(Ib)

5-Amino-4-heterocyclyl-1-pyridyl-pyrazole

Die Erfindung betrifft neue 5-Amino-4-heterocyclyl-1-pyridyl-pyrazole mehrere Verfahren zu ihrer Herstellung sowie ihre Verwendung als Herbizide.

Es ist bereits bekannt, daß bestimmte 5-Amino-1-phenyl-pyrazole, wie beispielsweise das 4-Cyano-5-propionamido-1-(2,4,6-trichlorphenyl)-pyrazol, herbizide Eigenschaften besitzen (vergl. z. B. DE-OS 32 26 513).

Die Wirksamkeit dieser vorbekannten Verbindungen ist jedoch, insbesondere bei niedrigen Aufwandmengen und -konzentrationen, nicht immer in allen Anwendungsbereichen völlig zufriedenstellend.

Es wurden neue 5-Amino-4-heterocyclyl-1-pyridyl-pyrazole der allgemeinen Formel (I),

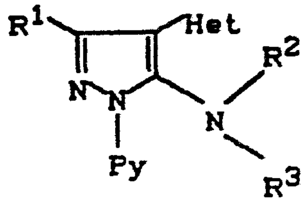

(I)

in welcher

$R^1$ für Wasserstoff oder Alkyl steht,

$R^2$ für Wasserstoff oder für einen Rest 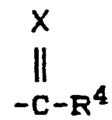 steht,

$R^3$ für Wasserstoff, für einen Rest 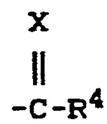, für Alkyl, Alkenyl oder Alkinyl steht,

wobei

$R^4$ für Wasserstoff, Alkyl, Alkoxy, Alkylthio, Alkoxyalkyl, Alkylthioalkyl, Halogenalkyl, Alkenyl, Alkinyl, für Alkylamino, für Dialkylamino, für gegebenenfalls substituiertes Cycloalkyl, für jeweils gegebenenfalls substituiertes Aryl, Aryloxy, Arylthio, Arylamino oder Aryloxyalkyl steht und

X für Sauerstoff oder Schwefel steht,

Py für gegebenenfalls substituiertes Pyridyl steht und

Het für einen gegebenenfalls substituierten gesättigten oder ungesättigten Heterocyclus steht,

gefunden.

Weiterhin wurde gefunden, daß man die neuen 5-Amino-4-heterocyclyl-1-pyridyl-pyrazole der allgemeinen Formel (I),

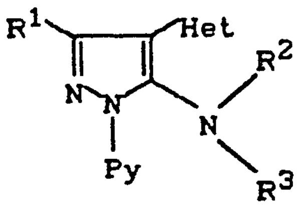

(I)

in welcher

$R^1$ für Wasserstoff oder Alkyl steht,

$R^2$ für Wasserstoff oder für einen Rest $-\overset{X}{\overset{\|}{C}}-R^4$ steht,

$R^3$ für Wasserstoff, für einen Rest $-\overset{X}{\overset{\|}{C}}-R^4$, für Alkyl, Alkenyl oder Alkinyl steht,

wobei

$R^4$ für Wasserstoff, Alkyl, Alkoxy, Alkylthio, Alkoxyalkyl, Alkylthioalkyl, Halogenalkyl, Alkenyl, Alkinyl, für Alkylamino, für Dialkylamino, für gegebenenfalls substituiertes Cycloalkyl, für jeweils gegebenenfalls substituiertes Aryl, Aryloxy, Arylthio, Arylamino oder Aryloxyalkyl steht und

X für Sauerstoff oder Schwefel steht,

Py für gegebenenfalls substituiertes Pyridyl steht und

Het für einen gegebenenfalls substituierten gesättigten oder ungesättigten Heterocyclus steht,

erhält, wenn man

(a) Pyridylhydrazine der Formel (II),

$$Py\text{-}NH\text{-}NH_2 \quad (II)$$

in welcher

Py die oben angegebene Bedeutung hat

mit Acrylnitril-Derivaten der Formel (III),

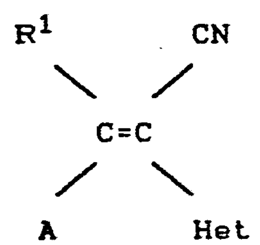

(III)

in welcher

$R^1$ und Het die oben angegebene Bedeutung haben und

A für Halogen, Hydroxy, Alkoxy oder Dialkylamino steht,

zunächst in einer 1.Stufe, gegebenenfalls in Gegenwart eines Verdünnungsmittels sowie gegebenenfalls in Gegenwart eines Reaktionshilfsmittels, umsetzt zu den Pyridylhydrazin-Derivaten der Formel (IV),

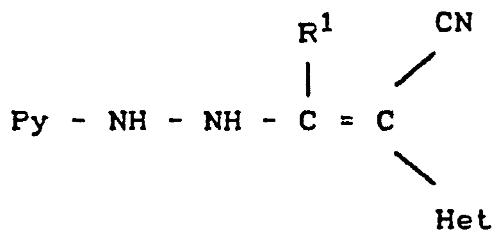

(IV)

in welcher

Py, $R^1$ und Het die oben angegebene Bedeutung haben,

und diese in einer 2. Stufe, gegebenenfalls in Gegenwart eines Verdünnungsmittels und gegebenenfalls in Gegenwart eines sauren Katalysators, cyclisiert zu den 5-Amino-4-heterocyclyl-1-pyridylpyrazolen der Formel (Ia),

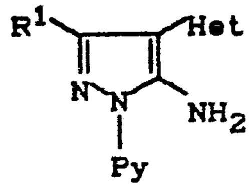

(Ia)

in welcher

$R^1$, Het und Py die oben angegebene Bedeutung haben,

oder wenn man

(b) die erfindungsgemäßen 5-Amino-4-heterocyclyl-1-pyridyl-pyrazole der Formel (Ia)

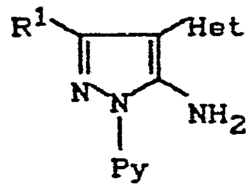

(Ia)

in welcher

$R^1$, Het und Py die oben angegebene Bedeutung haben,

mit Verbindungen der Formel (V),

$$R^{3-1} - A^1 \quad (V)$$

in welcher

$R^{3-1}$ für Alkyl, Alkenyl, Alkinyl oder einen Rest $-\overset{X}{\overset{\|}{C}}-R^4$ steht, wobei X und $R^4$ die oben angegebene Bedeutung haben und

$A^1$ für eine elektronenziehende Abgangsgruppe steht,

gegebenenfalls in Gegenwart eines Verdünnungsmittels und gegebenenfalls in Gegenwart eines Säurebindemittels, umsetzt zu den am Stickstoff alkylierten bzw. acylierten 5-Amino-4-heterocyclyl-1-pyridyl-pyrazolen der Formel (Ib),

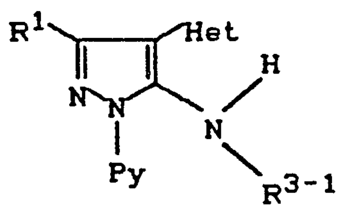

(Ib)

in welcher

$R^1$, $R^{3-1}$, Het und Py die oben angegebene Bedeutung haben,

oder wenn man

c) die erfindungsgemäßen 5-Amino-4-heterocyclyl-1-pyridyl-pyrazole der Formel (Ia),

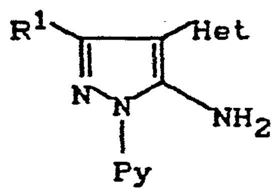

(Ia)

in welcher

$R^1$, Py und Het die oben angegebene Bedeutung haben,

mit Iso(thio)cyanaten der Formel (VI),

$$R^{4-1} - N = C = X \qquad (VI)$$

in welcher

$R^{4-1}$ für Alkyl oder für gegebenenfalls substituiertes Aryl steht und

X für Sauerstoff oder Schwefel steht,

gegebenenfalls in Gegenwart eines Verdünnungsmittels und gegebenenfalls in Gegenwart eines Reaktionshilfsmittels umsetzt zu den 5-Amino-4-heterocyclyl-1-pyridyl-pyrazolen der Formel (Ic),

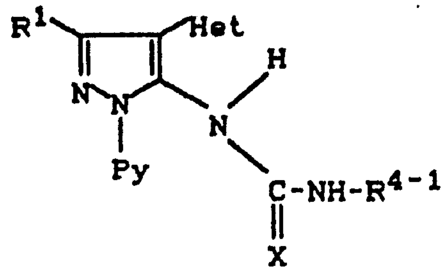

(Ic)

in welcher

$R^1$, $R^{4-1}$, Py, Het und X die oben angegebene Bedeutung haben.

Schließlich wurde gefunden, daß die neuen 5-Amino-4-heterocyclyl-1-pyridyl-pyrazole der Formel (I) herbizide Eigenschaften besitzen.

Überraschenderweise zeigen die erfindungsgemäßen 5-Amino-4-heterocyclyl-1-pyridyl-pyrazole der Formel (I) eine erheblich bessere herbizide Wirksamkeit als die aus dem Stand der Technik bekannten 5-Amino-1-phenyl-pyrazole, wie beispielsweise das 4-Cyano-5-propionamido-1-(2,4,6-trichlorphenyl)-pyrazol, welche chemisch und wirkungsmäßig naheliegende Verbindungen sind.

Die erfindungsgemäßen 5-Amino-4-heterocyclyl-1-pyridyl-pyrazole sind durch die Formel (I) allgemein definiert.

Bevorzugt sind Verbindungen der Formel (I), bei welchen

$R^1$ für Wasserstoff oder für geradkettiges oder verzweigtes Alkyl mit 1 bis 8 Kohlenstoffatomen steht,

$R^2$ für Wasserstoff oder für einen Rest $-\underset{\underset{X}{\|}}{C}-R^4$ steht,

$R^3$ für Wasserstoff, für einen Rest $-\overset{\overset{X}{\|}}{C}-R^4$, für geradkettiges oder verzweigtes Alkyl mit 1 bis 4 Kohlenstoffatomen oder für jeweils geradkettiges oder verzweigtes Alkenyl oder Alkinyl mit jeweils 2 bis 4 Kohlenstoffatomen steht, wobei

$R^4$ für Wasserstoff, für jeweils geradkettiges oder verzweigtes Alkyl, Alkoxy, Alkylthio, Alkoxyalkyl oder Alkylthioalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für jeweils geradkettiges oder verzweigtes Alkylamino oder Dialkylamino mit jeweils 1 bis 4 Kohlenstoffatomen in den einzelnen Alkylteilen, für gegebenenfalls einfach oder mehrfach, gleich oder verschieden substituiertes Cycloalkyl steht, wobei als Substituenten ausgewählt sind: Halogen, jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen; für jeweils gegebenenfalls substituiertes Phenyl, Phenoxy, Phenylthio, Phenylamino oder Phenyloxyalkyl, wobei als Phenyl-Substituenten jeweils in Frage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen in den Alkylteilen, jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen und

X für Sauerstoff oder Schwefel steht,

Py für jeweils gegebenenfalls einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy, Alkoxycarbonyl mit jeweils 1 bis 4 Kohlenstoffatomen in den einzelnen Alkylteilen, jeweils geradkettiges oder verzweigtes Halogenalkyl oder Halogenalkoxy mit 1 bis 4 Kohlenstoffatomen und 1 bis 9 gleichen oder verschiedenen Halogenatomen oder ein Rest $-S(O)_n-R^5$,

wobei

$R^5$ für jeweils geradkettiges oder verzweigtes Alkyl, Alkylamino oder Dialkylamino mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen oder für die unsubstituierte Aminogruppe steht und

n für eine Zahl 0, 1 oder 2 steht, und

Het für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschieden Heteroatome aus der Gruppe Stickstoff, Sauerstoff und Schwefel enthält und der über ein Kohlenstoff- oder ein Stickstoffatom verknüpft sein kann, wobei als Substituenten in Frage kommen: Halogen, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen.

Besonders bevorzugt sind Verbindungen der Formel (I), bei welchen

$R^1$ für Wasserstoff, Methyl, Ethyl, n- oder i-Propyl, n-, i-, s- oder t-Butyl steht,

$R^2$ für Wasserstoff, für einen Rest $-\overset{\overset{X}{\|}}{C}-R^4$ steht,

$R^3$ für Wasserstoff, für einen Rest $-\underset{\underset{X}{\|}}{C}-R^4$, für Methyl, Ethyl, n- und i-Propyl, n-, i-, s- und t-Butyl, für Allyl oder Propargyl steht, wobei

$R^4$ für Wasserstoff, Methyl, Ethyl, n- und i-Propyl, Methoxy, Ethoxy, Methylthio, Ethylthio, Methoxymethyl, Ethoxymethyl, Methoxyethyl, Ethoxyethyl, Methylthiomethyl, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 3-Chlorpropyl, 2-Bromethyl, Heptafluor-n-propyl, Allyl, Propargyl, für Methylamino, Ethylamino, Dimethylamino, Diethylamino, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenoxymethyl, Phenylthio oder Phenylamino steht,

X für Sauerstoff oder Schwefel steht,

Py für jeweils gegebenenfalls ein- bis vierfach, gleich oder verschieden substituiertes 2-Pyridyl, 3-Pyridyl oder 4-Pyridyl steht, wobei als Substituenten jeweils infrage kommen:

Cyano, Nitro, Fluor, Chlor, Brom, Iod, Methyl, Ethyl, n- und i-Propyl, n-, i-, s- und t- Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy,Trichlormethoxy, Dichlorfluormethoxy, Difluorchlormethoxy,Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^5$, wobei

$R^5$ für Methyl, Ethyl, Amino, Methylamino, Ethylamino, Dimethylamino, Diethylamino, Fluordichlormethyl, Difluorchlormethyl, Difluormethyl, Tetrafluorethyl, Trifluorchlorethyl, Trifluormethyl steht und

n für eine Zahl 0, 1 oder 2 steht, und

0205024

Het für einen gegebenenfalls ein- bis dreifach, gleich oder verschieden durch Fluor, Chlor, Brom, Nitro, Methyl, Ethyl, n- und i-Propyl, Methoxy, Ethoxy, n- oder i-Propoxy, Methylthio, Ethylthio, n- oder i- Propylthio oder Trifluormethyl substituierten Heterocyclus der Formel

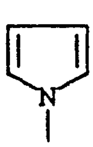 ; 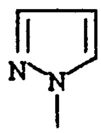 ; 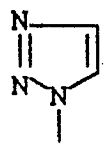 ; 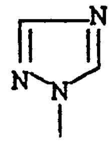 ; 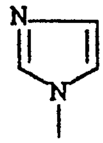 ; 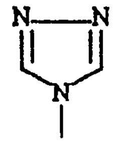 ;

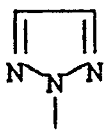 ; 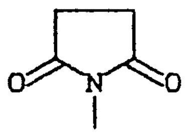 ; 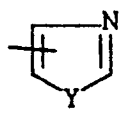 ; 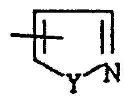 ; 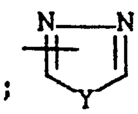 ;

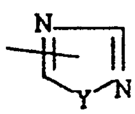 ; 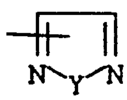 ; 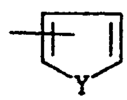 ; 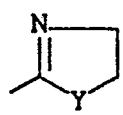 ; 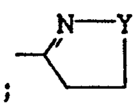 ;

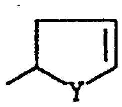 ; 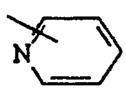 ; 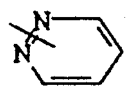 ; 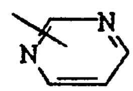 ; 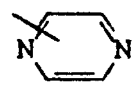 ;

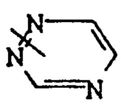 ; 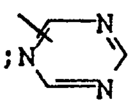 ; 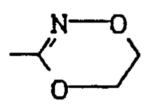 ; 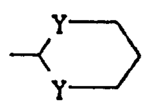 oder 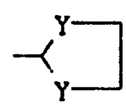 ;

steht, wobei

Y jeweils für Sauerstoff oder Schwefel oder einen N-Alkyl-Rest mit 1 bis 4 Kohlenstoffatomen steht.

Ganz besonders bevorzugt sind diejenigen Verbindungen der Formel (I), in denen $R^1$, $R^2$, $R^3$, $R^4$, $R^5$, Py, X und n die oben angegebene, besonders bevorzugte Bedeutung haben und

Het für einen gegebenenfalls ein- bis dreifach, gleich oder verschieden durch Fluor, Chlor, Brom, Nitro, Methyl, Ethyl, n- und i-Propyl, Methoxy, Ethoxy, n- oder i-Propoxy, Methylthio, Ethylthio, n- oder i- Propylthio oder Trifluormethyl substituierten Heterocyclus der Formel

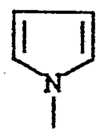 ; 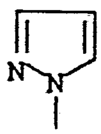 ; 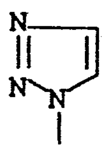 ; 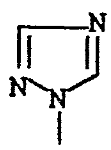 ; 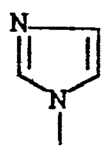 ; 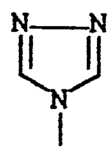 oder

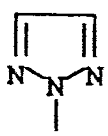 steht.

Im einzelnen seien außer den bei den Herstellungsbeispielen genannten Verbindungen die folgenden 5-Amino-4-heterocyclyl-1-pyridyl-pyrazole der allgemeinen Formel (I) genannt:

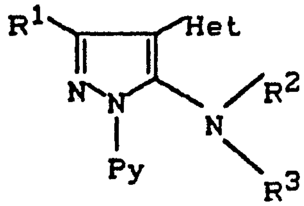

(I)

Tabelle 1

| $R^1$ | $R^2$ | $R^3$ | Py | Het |
|---|---|---|---|---|
| H | H | -CO-H | 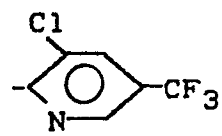 | 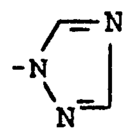 |
| H | H | $-CO-CH_3$ | 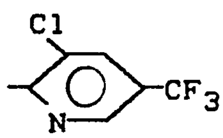 | 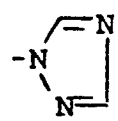 |
| H | H | $-CO-C_2H_5$ | 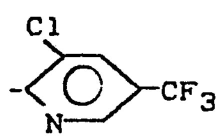 | 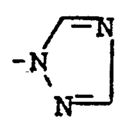 |
| H | H | $-CO-C_3H_7$ | 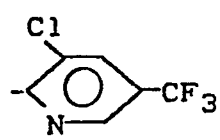 | 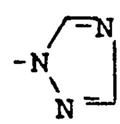 |
| H | H | $-CO-N(CH_3)_2$ | 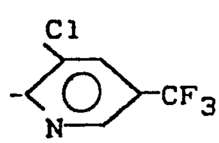 | 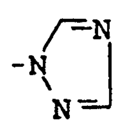 |

<u>Tabelle 1</u> (Fortsetzung)

| $R^1$ | $R^2$ | $R^3$ | Py | Het |
|---|---|---|---|---|
| H | H | $-CO-OCH_3$ | 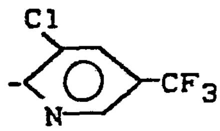 | 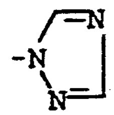 |
| H | H | $-CO-O-C_6H_5$ | 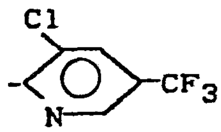 | 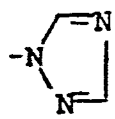 |
| H | H | $-CO-NHCH_3$ | 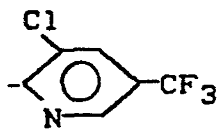 | 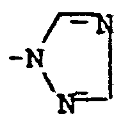 |
| H | H | $-CS-CH_3$ | 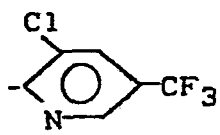 | 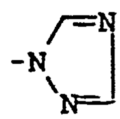 |
| H | H | $-CS-C_2H_5$ | 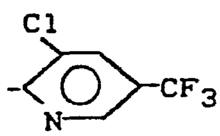 | 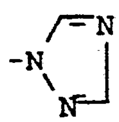 |
| H | H | H | 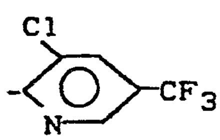 | 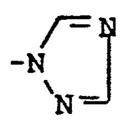 |

Tabelle 1 (Fortsetzung)

| $R^1$ | $R^2$ | $R^3$ | Py | Het |
|---|---|---|---|---|
| H | H | H | 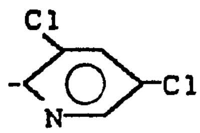 | 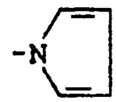 |
| H | H | -CO-H | 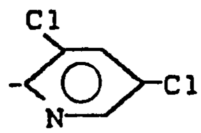 | 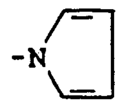 |
| H | H | $-CO-CH_3$ | 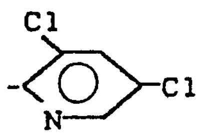 | 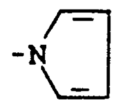 |
| H | H | $-CO-C_2H_5$ | 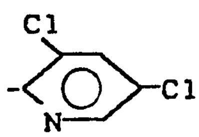 | 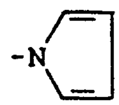 |
| H | H | H | 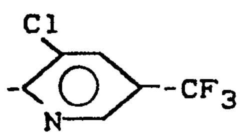 | 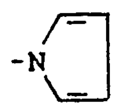 |
| H | H | -CO-H | 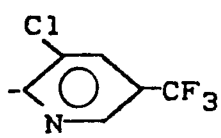 | 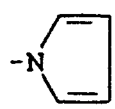 |

Tabelle 1 (Fortsetzung)

| $R^1$ | $R^2$ | $R^3$ | Py | Het |
|---|---|---|---|---|
| H | H | $-CO-CH_3$ | 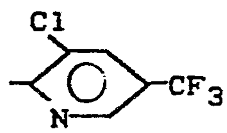 | 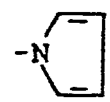 |
| H | H | $-CO-C_2H_5$ | 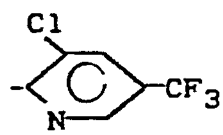 | 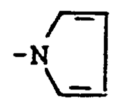 |
| H | H | H | 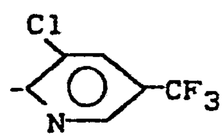 | 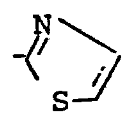 |
| H | H | $-CO-H$ | 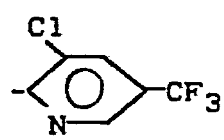 | 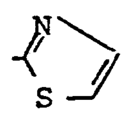 |
| H | H | $-CO-CH_3$ | 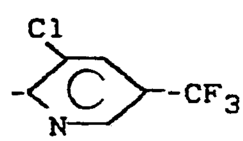 | 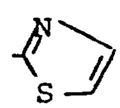 |
| H | H | $-CO-C_2H_5$ | 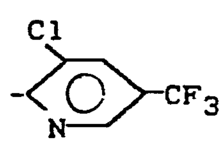 | 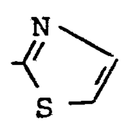 |

Tabelle 1 (Fortsetzung)

| $R^1$ | $R^2$ | $R^3$ | Py | Het |
|---|---|---|---|---|
| H | H | H | 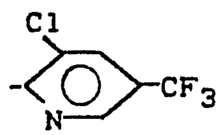 | 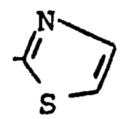 |
| H | H | $-CO-CH_3$ | 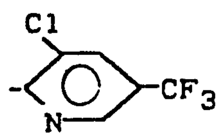 | 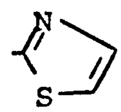 |
| H | H | $-CO-C_2H_5$ | 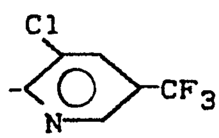 | 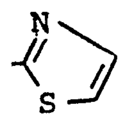 |

Tabelle 1 (Fortsetzung)

| $R^1$ | $R^2$ | $R^3$ | Py | Het |
|---|---|---|---|---|
| H | H | $-CO-CH_3$ | 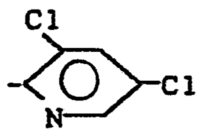 | 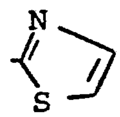 |
| H | H | $-CO-C_2H_5$ | 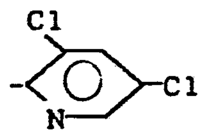 | 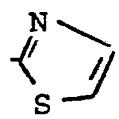 |
| H | H | H | 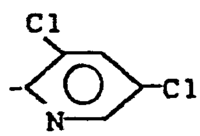 | 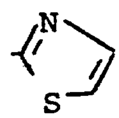 |
| H | H | -CO-H | 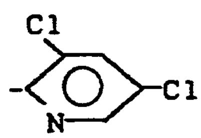 | 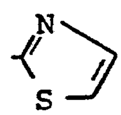 |

<u>Tabelle 1</u> (Fortsetzung)

| $R^1$ | $R^2$ | $R^3$ | Py | Het |
|---|---|---|---|---|
| H | H | H | 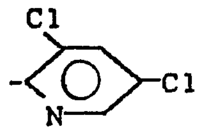 | 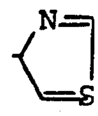 |
| H | H | $-CO-C_2H_5$ | 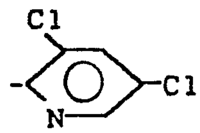 | 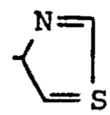 |
| H | H | H | 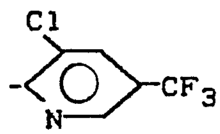 | 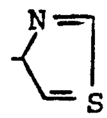 |
| H | H | $-CO-C_2H_5$ | 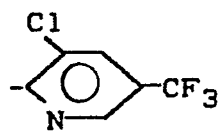 | 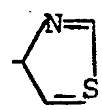 |
| H | H | H | 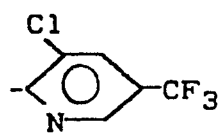 | 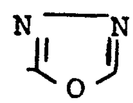 |
| H | H | $-CO-C_2H_5$ | 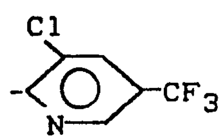 | 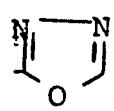 |

Tabelle 1 (Fortsetzung)

| $R^1$ | $R^2$ | $R^3$ | Py | Het |
|---|---|---|---|---|
| H | H | H | 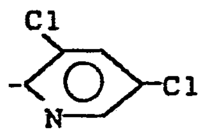 | 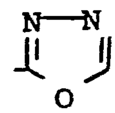 |
| H | H | $-CO-C_2H_5$ | 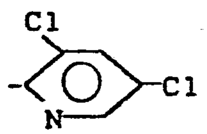 | 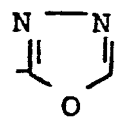 |
| H | H | H | 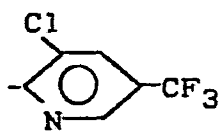 | 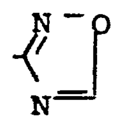 |
| H | H | $-CO-C_2H_5$ | 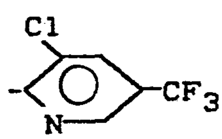 | 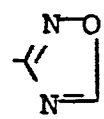 |
| H | H | H | 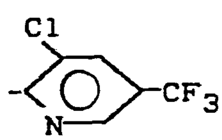 | 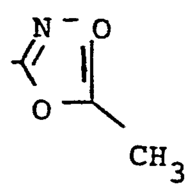 |

<u>Tabelle 1</u> (Fortsetzung)

| $R^1$ | $R^2$ | $R^3$ | Py | Het |
|---|---|---|---|---|
| H | H | $-CO-C_2H_5$ | 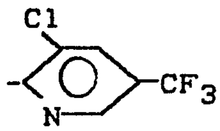 | 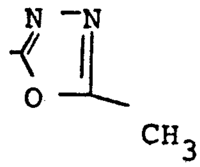 |
| H | H | H | 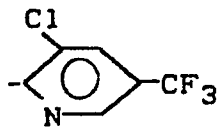 | 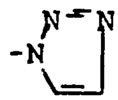 |
| H | H | $-CO-C_2H_5$ | 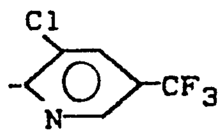 | 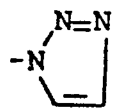 |
| H | H | H | 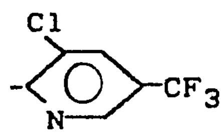 | 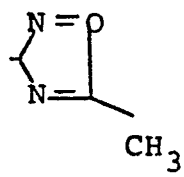 |
| H | H | $-CO-C_2H_5$ | 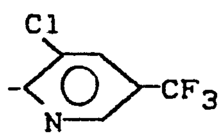 | 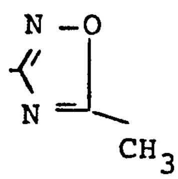 |

Tabelle 1 (Fortsetzung)

| $R^1$ | $R^2$ | $R^3$ | Py | Het |
|---|---|---|---|---|
| H | H | H | 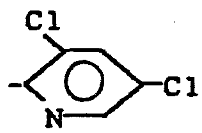 | 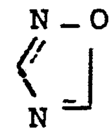 |
| H | H | $-CO-C_2H_5$ | 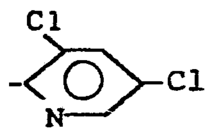 | 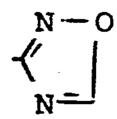 |
| H | H | H | 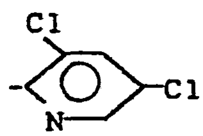 | 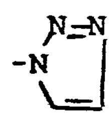 |
| H | H | $-CO-C_2H_5$ | 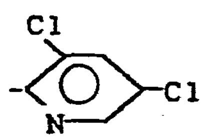 | 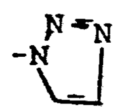 |
| $CH_3$ | H | H | 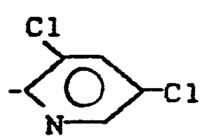 | 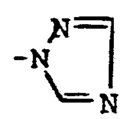 |
| $CH_3$ | H | -CO-H | 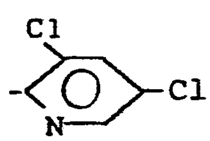 | 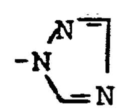 |
| $CH_3$ | H | $-CO-CH_3$ | 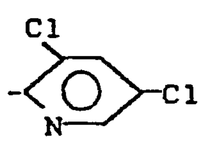 | 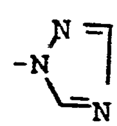 |

<u>Tabelle 1</u> (Fortsetzung)

| $R^1$ | $R^2$ | $R^3$ | Py | Het |
|---|---|---|---|---|
| $CH_3$ | H | $-CO-C_2H_5$ | 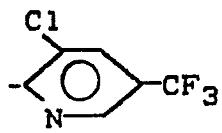 | 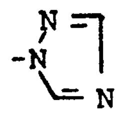 |
| $CH_3$ | H | H | 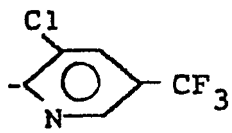 | 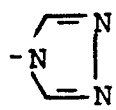 |
| $CH_3$ | H | $-CO-C_2H_5$ | 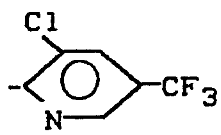 | 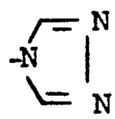 |
| $CH_3$ | H | H | 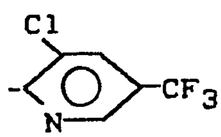 | 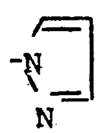 |
| $CH_3$ | H | $CO-CH_3$ | 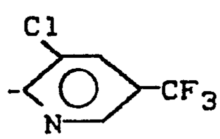 | 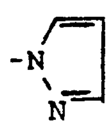 |
| $CH_3$ | H | $-CO-C_2H_5$ | 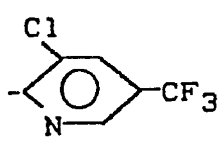 | 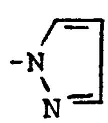 |

Tabelle 1 (Fortsetzung)

| $R^1$ | $R^2$ | $R^3$ | Py | Het |
|---|---|---|---|---|
| $CH_3$ | H | $-CO-OCH_3$ | 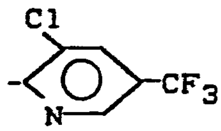 | 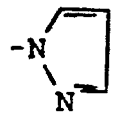 |
| $CH_3$ | H | H | 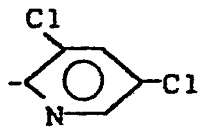 | 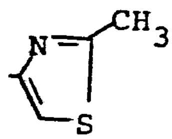 |
| $CH_3$ | H | $-CO-CH_3$ | 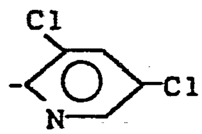 | 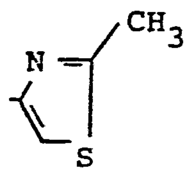 |
| $CH_3$ | H | $-CO-C_2H_5$ | 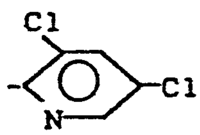 | 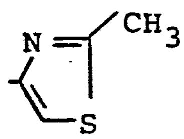 |
| $CH_3$ | H | H | 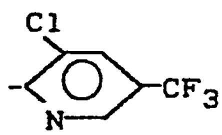 | 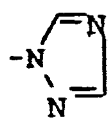 |
| $CH_3$ | H | $-CO-C_2H_5$ | 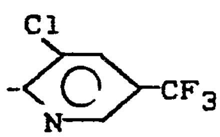 | 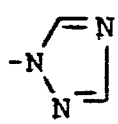 |

Verwendet man beispielsweise 3,5-Dichlor-2-hydrazino-pyridin und 3-Dimethylamino-2-[1,2,4-triazol-1-yl]-acrylnitril-Hydrochlorid, so läßt sich der Reaktionsablauf des erfindungsgemäßen Verfahrens (a) durch das folgende Formelschema darstellen:

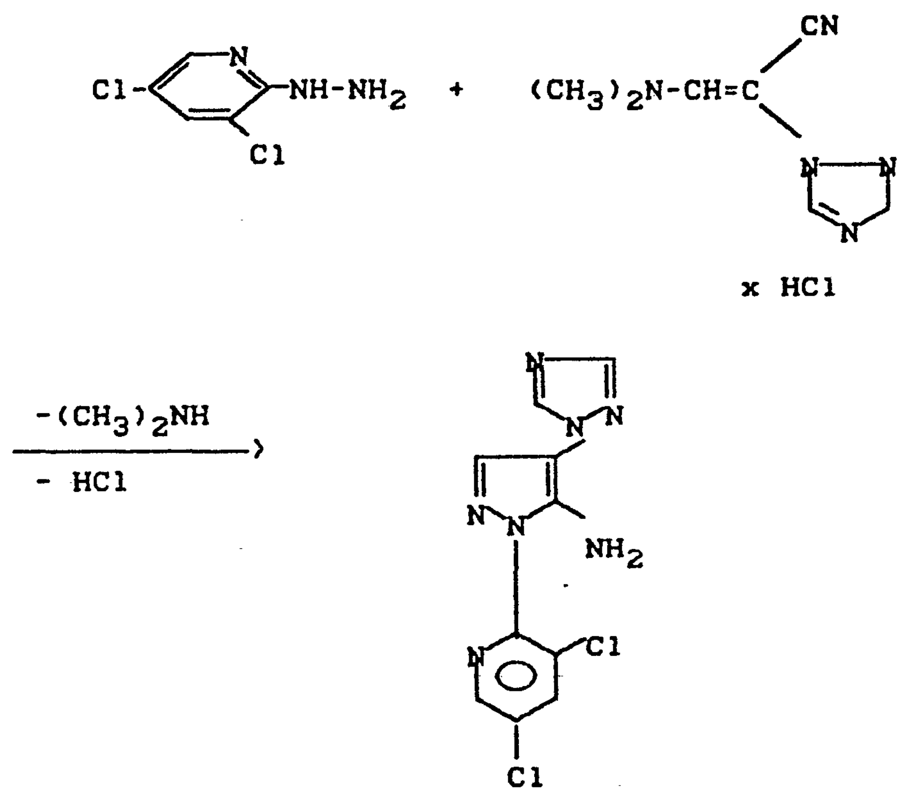


Verwendet man beispielsweise 1-(3,5-Dichlor-2-pyridyl)-5-amino-4-(1,2,4-triazol-1-yl)-pyrazol und Propionylchlorid als Ausgangsstoffe, so läßt sich der Reaktionsablauf des erfindungsgemäßen Verfahrens (b) durch das folgende Formelschema darstellen:

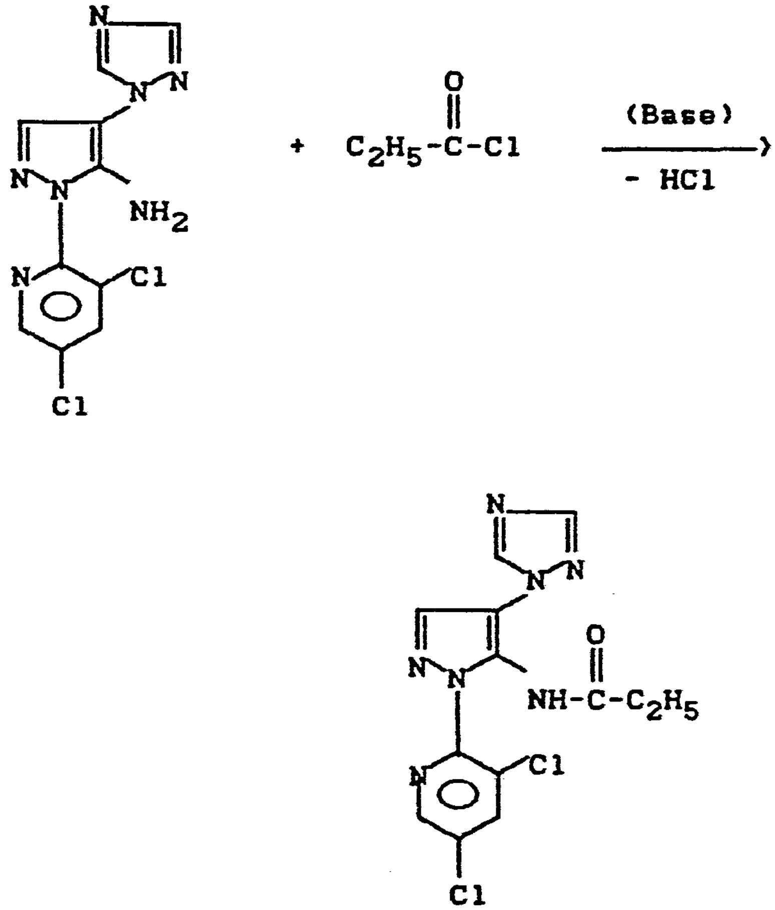


Verwendet man beispielsweise 1-(3,5-Dichlor-2-pyridyl)-5-amino-4-(1,2,4-triazol-1-yl)-pyrazol und Methylisocyanat als Ausgangsstoffe, so läßt sich der Reaktionsverlauf des erfindungsgemäßen Verfahrens (c) durch das folgende Formelschema darstellen:

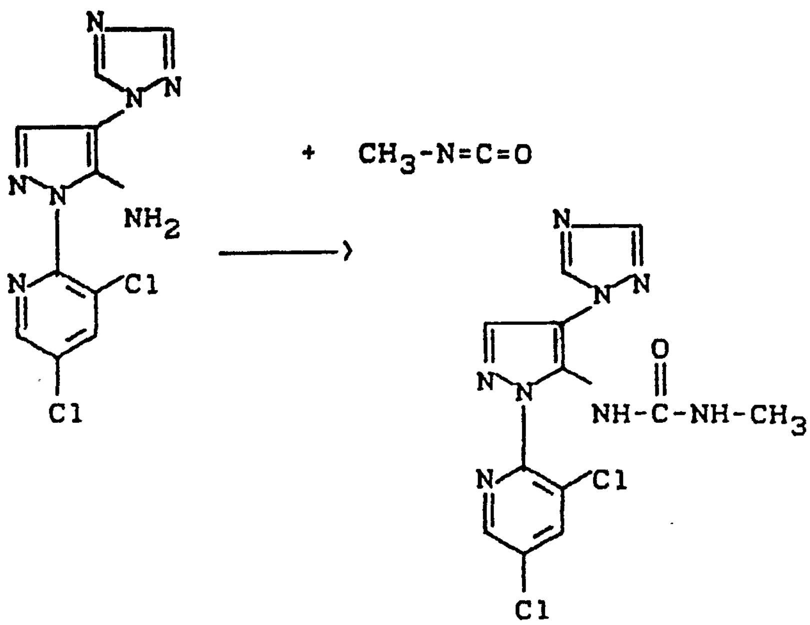

Die zur Durchführung des erfindungsgemäßen Verfahrens (a) als Ausgangsstoffe benötigten Pyridylhydrazine sind durch die Formel (II) allgemein definiert. In dieser Formel (II) steht Py vorzugsweise für diejenigen Substituenten, die bereits bei der Beschreibung der erfindungsgemäßen Stoffe der Formel (I) als bevorzugt für diesen Rest genannt wurden.

Die Pyridylhydrazine der Formel (II) sind größtenteils bekannt (vergl. z. B. US-PS 4.127.575; US-PS 3.609.158; DE-OS 25 58 399; J. Chem. Soc C, 167-174 (1971)) oder lassen sich nach prinzipiell bekannten Verfahren in einfacher Weise herstellen (vergl.: z. B. Houben-Weyl 'Methoden der organischen Chemie', Band X/2, S. 203,

Thieme Verlag Stuttgart 1967), indem man beispielsweise die bekannten Pyridylamine der Formel (VII),

$$Py\text{-}NH_2 \qquad (VII)$$

in welcher

Py die oben angegebene Bedeutung hat,

mit Natriumnitrit, in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20°C und +80°C umsetzt.

Die weiterhin zur Durchführung des Herstellungsverfahrens (a) als Ausgangsstoffe benötigten Acrylnitril-Derivate sind durch die Formel (III) allgemein definiert. In dieser Formel (III) stehen $R^1$ und Het vorzugsweise für diejenigen Substituenten, die bereits im Zusammenhang mit der Beschreibung der erfindungsgemäßen Stoffe der Formel (I) als bevorzugt für diese Substituenten genannt wurden. A steht vorzugsweise für Chlor, Brom, Methoxy, Ethoxy oder Dimethylamino.

Die Acrylnitril-Derivate der Formel (III) sind noch nicht bekannt. Sie sind jedoch Gegenstand einer eigenen vorgängigen Patentanmeldung (DE-P 34 23 101 vom

22.06.84). Man erhält sie, wenn man Acetonitril-Derivate der Formel (VIII),

$Het-CH_2-CN$ (VIII)

in welcher

Het die oben angegebene Bedeutung hat

entweder mit Orthoestern der Formel (IX),

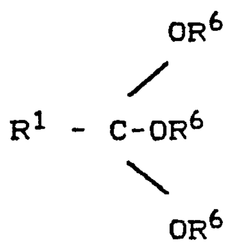

(IX)

in welcher

$R^1$ die oben angegebene Bedeutung hat und

$R^6$ für Alkyl, insbesondere für Methyl oder Ethyl steht,

oder mit Amidacetalen der Formel (X),

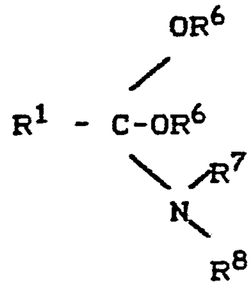

(X)

in welcher

$R^1$ und $R^6$ die oben angegebene Bedeutung haben und

$R^7$ und $R^8$ unabhängig voneinander für Alkyl, insbesondere für Methyl stehen,

gegebenenfalls in Gegenwart eines Verdünnungsmittels, wie beispielsweise Toluol, bei Temperaturen zwischen +80 °C und +200 °C umsetzt,

oder mit Estern der Formel (XI),

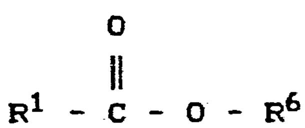

(XI)

in welcher

$R^1$ und $R^6$ die oben angegebene Bedeutung haben,

gegebenenfalls in Gegenwart eines Verdünnungsmittels, wie beispielsweise Methanol oder Ethanol, und gegebenenfalls in Gegenwart eines basischen Katalysators, wie beispielsweise Natriummethylat oder -ethylat, bei Temperaturen zwischen 0 °C und 100 °C umsetzt und die so erhältlichen Hydroxyverbindungen der Formel (IIIa),

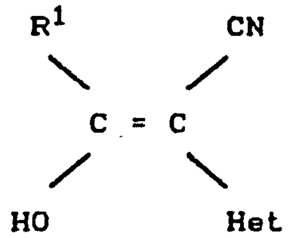

(IIIa)

in welcher

$R^1$ und Het die oben angegebene Bedeutung haben,

in üblicher Weise mit Halogenierungsmitteln, wie Phosphorpentachlorid, Thionylchlorid oder Phosphortribromid, bei Temperaturen zwischen 0 °C und + 100 °C substituiert.

Die Pyridylamine der Formel (VII) und die Acetonitril-Derivate der Formel (VIII) sind bekannt [vergl. z. B. Zh. org. Khim. 18, 463 (1982); C.A. 96, 181213x; DE- OS 31 29 429; Ber. dtsch. chem. Ges. 27, 3151 (1894)] oder lassen sich nach bekannten Verfahren in einfacher analoger Weise herstellen.

Die Orthoester der Formel (IX), die Amidacetale der Formel (X) und die Ester der Formel (XI) sind allgemein bekannte Verbindungen der organischen Chemie.

Die zur Durchführung der erfindungsgemäßen Verfahren (b) und (c) als Ausgangsstoffe benötigten 5-Amino-4-heterocyclyl-1-pyridyl-pyrazole sind durch die Formel (Ia) allgemein definiert. In der Formel (Ia) stehen $R^1$, Py und Het vorzugsweise für diejenigen Reste, die schon bei der Beschreibung der erfindungsgemäßen Stoffe der Formel (I) als bevorzugt für diese Substituenten genannt wurden.

Die 5-Amino-4-heterocyclyl-1-pyridyl-pyrazole der Formel (Ia) sind erfindungsgemäße Verbindungen und erhältlich nach Herstellungsverfahren (a).

Die weiterhin zur Durchführung des Herstellungsverfahrens (b) als Ausgangsstoffe benötigten Verbindungen sind durch die Formel (V) allgemein definiert. In dieser Formel (V) steht $R^{3-1}$ vorzugsweise für jeweils geradkettiges oder verzweigtes Alkyl mit 1 bis 4 Kohlenstoffatomen, für jeweils geradkettiges oder verzweigtes Alkenyl oder Alkinyl mit jeweils 2 bis 4 Kohlenstoffatomen oder für einen Rest $-\underset{\underset{X}{\|}}{C}-R^4$, wobei X und $R^4$ vorzugsweise für diejenigen Reste stehen, die schon bei der Beschreibung der erfindungsgemäßen Stoffe der Formel (I) als bevorzugt für diese Reste genannt wurden.

$A^1$ steht vorzugsweise für Chlor, Brom oder Iod, p-Toluolsulfonyloxy, Alkoxysulfonyloxy oder Acyloxy. Die Verbindungen der Formel (V) sind allgemein bekannte Verbindungen der organischen Chemie.

Außerdem können die nach Verfahren (b) hergestellten 5-Amino-4-heterocyclyl-1-pyridyl-pyrazole der Formel (Ib) erneut als Ausgangsstoffe bei Herstellungsverfahren (b) eingesetzt werden. Auf diese Weise erhält man beispielsweise gemischt Acyl-alkyl-substituierte Verbindungen.

Die weiterhin zur Durchführung des Herstellungsverfahrens (c) als Ausgangsstoffe benötigten Iso(thio)cyanate sind durch die Formel (VI) allgemein definiert. In dieser Formel (VI) steht X vorzugsweise für Sauerstoff oder Schwefel und $R^{4-1}$ steht vorzugsweise für geradkettiges oder verzweigtes Alkyl mit 1 bis 4 Kohlenstoffatomen, oder für gegebenenfalls ein- bis dreifach, gleich oder verschieden substituiertes Phenyl, wobei als Substituenten infrage kommen: Halogen, jeweils geradkettiges oder verzweigtes Alkyl oder Alkoxy mit jeweils 1 bis 4 Kohlenstoffatomen oder geradkettiges oder verzweigtes Halogenalkyl mit 1 bis 4 Kohlenstoffatomen und mit 1 bis 9 gleichen oder verschiedenen Halogenatomen. $R^{4-1}$ steht insbesondere für Methyl, Ethyl und gegebenenfalls für ein- bis dreifach, gleich oder verschieden durch Fluor, Chlor, Methyl, Methoxy oder Trifluormethyl substituiertes Phenyl.

Die Iso(thio)cyanate der Formel (VI) sind ebenfalls allgemein bekannte Verbindungen der organischen Chemie.

Als Verdünnungsmittel zur Durchführung des Herstellungsverfahrens (a) kommen sowohl für die 1. als auch für die 2. Reaktionsstufe inerte organische Lösungsmittel in

Frage. Vorzugsweise verwendet man Alkohole, wie Methanol, Ethanol, Propanol, Butanol, Ethylenglykol oder Ethylenglykolomonomethyl- oder -ethylether, oder Ether wie Ethylenglykoldimethylether.

Als Reaktionshilfsmittel zur Durchführung der 1. Stufe des Herstellungsverfahrens (a) kommen organische oder anorganische Säuren infrage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe des Herstellungsverfahrens (a) in gewissen Bereichen variiert werden . Im allgemeinen arbeitet man zwischen -30 °C und +50 °C, vorzugsweise zwischen -20 °C und +20 °C.

Als Katalysatoren zur Durchführung der 2. Stufe des Herstellungsverfahrens (a) kommen alle üblicherweise verwendbaren Säuren, wie beispielsweise Schwefelsäure oder Phosphorsäure, in Frage.

Das Herstellungsverfahren (a) kann auch direkt in einem Reaktionsschritt ohne Isolierung der Zwischenprodukte der Formel (IV) durchgeführt werden.

Die Reaktionstemperaturen können bei der Durchführung der 2. Stufe des Herstellungsverfahrens (a) ebenso wie bei der einstufigen Reaktionsführung in einem größeren

Bereich variiert werden. Im allgemeinen arbeitet man zwischen 0 °C und +200 °C, vorzugsweise zwischen +50 °C und+150 °C.

Zur Durchführung des Herstellungsverfahrens (a) setzt man sowohl bei der einstufigen als auch bei der zweistufigen Reaktionsführung pro Mol Pyridylhydrazin der Formel (II) im allgemeinen 1,0 bis 3,0 Mol, vorzugsweise 1,0 bis 1,5 Mol an Acrylnitril-Derivat der Formel (III) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an saurem Katalysator ein.

Die Aufarbeitung und Isolierung der Reaktionsprodukte erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Zur Durchführung des Herstellungsverfahrens (b) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methyl-pyrrolidon oder Hexamethylphosphorsäuretriamid. Verwendet man Verbindungen der Formel (V) in flüssiger Form, so ist es auch möglich, diese in entsprechendem Überschuß als Verdünnungsmittel einzusetzen.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (b) in einem größeren Bereich variiert werden. Im allgemeinen arbeitet man zwischen -20 °C und + 150 °C, vorzugsweise zwischen 0 °C und 100 °C.

Zur Durchführung des Herstellungsverfahrens (b) setzt man pro Mol 5-Amino-4-heterocyclyl-1-pyridyl-pyrazol der Formel (Ia) im allgemeinen 1,0 bis 20,0 Mol, vorzugsweise 1,0 bis 15,0 Mol an Verbindung der Formel (V)

und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Ib) erfolgt in allgemein üblicher Art und Weise.

Zur Durchführung des erfindungsgemäßen Verfahrens (c) kommen als Verdünnungsmittel inerte organische Lösungsmittel in Frage. Vorzugsweise verwendet man die bei der Durchführung des Herstellungsverfahrens (b) genannten Lösungsmittel. Verwendet man Verbindungen der Formel (VI) in flüssiger Form, so ist es auch möglich, diese in entsprechenden Überschuß als Verdünnungsmittel einzusetzen.

Als Reaktionshilfsmittel zur Durchführung des Herstellungsverfahrens (c) kommen tertiäre Amine in Frage wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (c) in einem größeren Bereich variiert werden. Im allgemeinen arbeitet man zwischen -20°C und +150°C, vorzugsweise zwischen 0°C und 100°C.

Zur Durchführung des Herstellungsverfahrens (c) setzt man pro Mol 5-Amino-4-heterocyclyl-1-pyridyl-pyrazol der

Formel (Ia) im allgemeinen 1,0 bis 20,0 Mol, vorzugsweise 1,0 bis 15,0 Mol an Verbindung der Formel (VI) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Ic) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea,

Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-. Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg als selektive Unkrautbekämpfungsmittel von dikotylen Unkräutern in dikotylen und monokotylen Kulturen wie Soja und Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Steckmitteln können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine,z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische,pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 4-Amino-6-t-butyl-3-ethylthio-1,2,4-triazin-5(4H)-on; 2-[4-(3,5-Dichlorpyrid-2-yloxy)-phenoxy)-propionsäure-(2-benzyloxyethylester), -(trimethyl-

silylmethylester), oder -(2,2-diethoxyethylester); 2-(1-(Ethoxamino)-butyliden]-5-(2-ethylthiopropyl)-1,3-cyclohexandion, Methyl-5-(2,4-dichlorphenoxy)-2-nitrobenzoat; 3-Isopropyl-2,1,3-benzothiadiazin-4-on-2,2-dioxid; 2-{4-[[3-Chlor-5-(trifluormethyl)-2-pyridyl]-oxy]-phenoxy}-propansäure bzw. -propansäureethylester; 5-(2-Chlor-4-trifluormethylphenoxy)-2-nitrobenzoesäure; 6-Chlor-3-phenyl-pyridazin-4-yl-S-octylthiocarbonat; 2-Chlor-4-ethylamino-6-isopropylamino-1,3,5-triazin; 2-[5-Methyl-5-(1-methylethyl)-4-oxo-2-imidazolin-2-yl]- 3-chinolincarbonsäure sowie Ethyl-2-[[(4-chlor-6- methoxy-2-pyrimidinyl)-aminocarbonyl]-aminosufonyl]-benzoat sind von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach denm Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

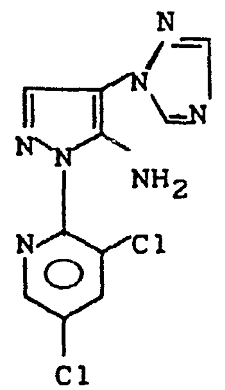

( Verfahren (a))

9g (0,05 Mol) 3,5-Dichlor-2-pyridylhydrazin und 10 g (0,05 Mol) 3-Dimethylamino-2-(1,2,4-triazol-1-yl)-acrylnitril-Hydrochlorid in 80 ml Ethanol werden für 5 Stunden unter Rückfluß erhitzt. Die erkaltete Reaktionsmischung wird in Wasser gegossen, abgesaugt, und getrocknet, dann in Diethylenglykoldimethylether aufgenommen, und weitere 4 Stunden auf 140 °C bis 145 °C erhitzt. Die erkaltete Reaktionsmischung wird wiederum in Wasser gegossen, gekühlt, abgesaugt und auf Ton getrocknet.

Man erhält 12 g (81 % der Theorie) an 5-Amino-1-(3,5-dichlor-2-pyridyl)-4-(1,2,4-triazol-1-yl)-pyrazol vom Schmelzpunkt 142 °C.

Herstellung der Ausgangsverbindungen:

a) 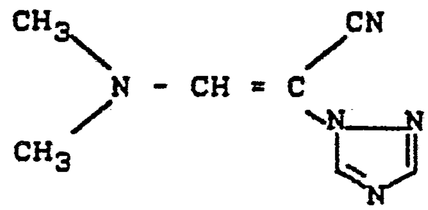 x HCl

108 g (1 Mol) 1,2,4-Triazol-1-yl-acetonitril und 160 g (1,35 Mol) N,N-Dimethylformamid-dimethylacetal werden 4 Stunden unter Rückfluß erhitzt; das entstandene Methanol wird abdestilliert bis die Innentemperatur auf 120 °C angestiegen ist; der erhaltene Rückstand wird mit 400 ml einer 20%igen ethanolischen Chlorwasserstofflösung versetzt. Der kristalline Niederschlag wird abgesaugt, mit wenig Ethanol nachgewaschen und auf Ton getrocknet.

Man erhält 163 g (82 % der Theorie) an 3-Dimethylamino-2-(1,2,4-triazol-1-yl)-acrylnitril-Hydrochlorid vom Schmelzpunkt 200 - 204°C

b) 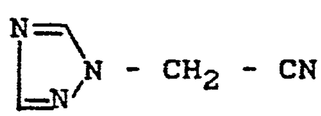

Zu einer Mischung aus 69 g (1 Mol) 1,2,4-Triazol, 150 g (1,08 Mol) gemahlenem Kaliumcarbonat und 500 ml Acetonitril gibt man tropfenweise unter Rühren bei 65-70 °C während 30 Minuten 76 g (1 Mol) Chloracetonitril, rührt nach beendeter Zugabe weitere 3 Stunden bei 70 °C, filtriert aus der erhaltenen Reaktionsmischung den unlöslichen Niederschlag ab, engt das Filtrat im Vakuum ein und destilliert den Rückstand im Hochvakuum.

Man erhält 75 g (69 % der Theorie) an 1,2,4-Triazol-1-yl-acetonitril vom Siedepunkt 85 °C bei 0,01 mbar, welches beim Erkalten erstarrt und einen Schmelzpunkt von 55 °C bis 58 °C besitzt.

In entsprechender Weise und gemäß den allgemeinen Herstellungsangaben erhält man die folgenden Verbindungen der allgemeinen Formel (I):

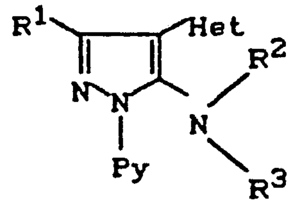

(I)

Tabelle 2:

| Bsp. Nr. | $R^1$ | $R^2$ | $R^3$ | Het | Py | Schmelzpunkt [°C] |
|---|---|---|---|---|---|---|
| 2 | H | H | $C_2H_5$-CO- | 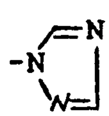 | 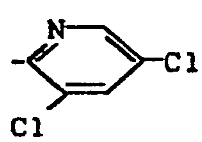 | 135 |

Anwendungsbeispiel:

In dem folgenden Anwendungsbeispiel wurde die nachstehend aufgeführte Verbindung als Vergleichssubstanz herangezogen:

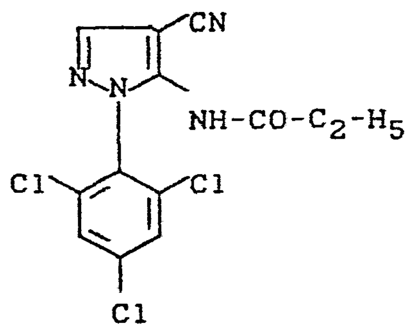

(A)

4-Cyano-5-propionamido-1-(2,4,6-trichlorphenyl)-pyrazol.

(Bekannt aus DE-OS 32 26 513)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Kulturpflanzenselektivität gegenüber der Vergleichssubstanz (A) zeigt in diesem Test z.B. die Verbindung gemäß den Herstellungsbeispielen 1 und 2.

## Patentansprüche

1. 5-Amino-4-heterocyclyl-1-pyridyl-pyrazole der Formel (I)

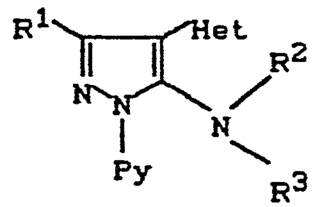

(I)

in welcher

$R^1$ für Wasserstoff oder Alkyl steht,

$R^2$ für Wasserstoff oder für einen Rest $-\overset{X}{\overset{\parallel}{C}}-R^4$ steht,

$R^3$ für Wasserstoff, für einen Rest $-\overset{X}{\overset{\parallel}{C}}-R^4$, für Alkyl, Alkenyl oder Alkinyl steht,

wobei

$R^4$ für Wasserstoff, Alkyl, Alkoxy, Alkylthio, Alkoxyalkyl, Alkylthioalkyl, Halogenalkyl, Alkenyl, Alkinyl, für Alkylamino für Dialkylamino, für gegebenenfalls substituiertes Cycloalkyl, für jeweils gegebenenfalls

substituiertes Aryl, Aryloxy, Arylthio, Arylamino oder Aryloxyalkyl steht und

X für Sauerstoff oder Schwefel steht,

Py für gegebenenfalls substituiertes Pyridyl steht und

Het für einen gegebenenfalls substituierten gesättigen oder ungesättigten Heterocyclus steht.

2. 5-Amino-4-heterocyclyl-1-pyridyl-pyrazole der Formel (I)

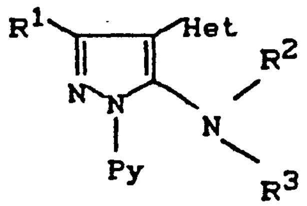

(I)

in welcher

$R^1$ für Wasserstoff oder für geradkettiges oder verzweigtes Alkyl mit 1 bis 8 Kohlenstoffatomen steht,

$R^2$ für Wasserstoff oder für einen Rest $-\underset{\underset{X}{\|}}{C}-R^4$ steht,

$R^3$ für Wasserstoff, für einen Rest $-\overset{X}{\overset{\|}{C}}-R^4$, für geradkettiges oder verzweigtes Alkyl mit 1 bis 4 Kohlenstoffatomen, oder für jeweils geradkettiges oder verzweigtes Alkenyl oder Alkinyl mit jeweils 2 bis 4 Kohlenstoffatomen steht, wobei

$R^4$ für Wasserstoff, für jeweils geradkettiges oder verzweigtes Alkyl, Alkoxy, Alkylthio, Alkoxyalkyl oder Alkylthioalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für jeweils geradkettiges oder verzweigtes Alkylamino oder Dialkylamino mit jeweils 1 bis 4 Kohlenstoffatomen in den einzelnen Alkylteilen, für gegebenenfalls einfach oder mehrfach, gleich oder verschieden substituiertes Cycloalkyl steht, wobei als Substituenten infrage kommen: Halogen, jeweils geradkettiges oder verzweigtes Alkyl mit 1 bis 4 Kohlenstoffatomen oder Halogenalkyl mit 1 bis 4 Kohlenstoffatomen und 1 bis 9 gleichen oder verschiedenen Halogenatomen; für jeweils gegebenenfalls substituiertes Phenyl, Phenoxy, Phenylthio, Phenylamino oder Phenyloxyalkyl, wobei als Phenyl- Substituenten jeweils infrage kommen: Halogen,

Cyano, Nitro, jeweils geradkettiges oder verzweigtes Alkyl, Alkoxy, Alkylthio oder Alkoxycarbonyl mit jeweils 1 bis 4 Kohlenstoffatomen in den Alkylteilen, jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen und

X für Sauerstoff oder Schwefel steht,

Py für jeweils gegebenenfalls einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen in den einzelnen Alkylteilen, jeweils geradkettiges oder verzweigtes Halogenalkyl oder Halogenalkoxy mit 1 bis 4 Kohlenstoffatomen und 1 bis 9 gleichen oder verschiedenen Halogenatomen oder ein Rest $-S(O)_n-R^5$,

wobei

$R^5$ für jeweils geradkettiges oder verzweigtes Alkyl, Alkylamino oder Dialkylamino mit jeweils 1 bis 4 Kohlenstoffatomen in den einzelnen Alkylteilen, für geradkettiges oder verzweigtes Halogenalkyl mit 1 bis 4 Kohlenstoffatomen und

1 bis 9 gleichen oder verschiedenen Halogenatomen oder für die unsubstituierte Aminogruppe steht und

n für eine Zahl 0, 1 oder 2 steht, und

Het für einen gegebenenfalls einfach oder mehrfach, gleich oder verschieden substituierten, gesättigten oder ungesättigten, fünf- oder sechsgliedrigen Heterocyclus steht, der ein bis drei gleiche oder verschieden Heteroatome aus der Gruppe Stickstoff, Sauerstoff und Schwefel enthält und der über ein Kohlenstoff- oder ein Stickstoffatom verknüpft sein kann, wobei als Substituenten in Frage kommen: Halogen, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen.

3. 5-Amino-4-heterocyclyl-1-pyridyl-pyrazole der Formel (I)

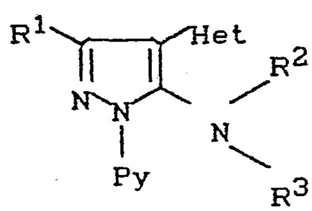

(I)

in welcher

$R^1$ für Wasserstoff, Methyl, Ethyl, n- oder i-Propyl, n-, i-, s- oder t-Butyl steht,

$R^2$ für Wasserstoff oder für einen $-\overset{X}{\overset{\|}{C}}-R^4$ steht,

$R^3$ für Wasserstoff, für einen Rest $-\overset{X}{\overset{\|}{C}}-R^4$, für Methyl, Ethyl, n- und i-Propyl, n-, i-, s- und t-Butyl, für Allyl oder Propargyl steht,

wobei

$R^4$ für Wasserstoff, Methyl, Ethyl, n- und i-Propyl, Methoxy, Ethoxy, Methylthio, Ethylthio, Methoxymethyl, Ethoxymethyl, Methoxyethyl, Ethoxymethyl, Ethoxyethyl, Methylthiomethyl, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 3-Chlorpropyl, 2-Bromethyl, Heptafluor-n-propyl, für Allyl, Propargyl, für Methylamino, Ethylamino, Dimethylamino, Diethylamino, für jeweils gegebenenfalls ein- bis vierfach, gleich oder verschieden durch Fluor, Chlor, Brom, Methyl oder Trifluormethyl substituiertes Cyclopropyl,

Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenoxymethyl, Phenylthio oder Phenylamino steht,

X für Sauerstoff oder Schwefel steht,

Py für jeweils gegebenenfalls ein- bis vierfach, gleich oder verschieden substituiertes 2-Pyridyl, 3-Pyridyl oder 4-Pyridyl steht, wobei als Substituenten jeweils infrage kommen:

Cyano, Nitro, Fluor, Chlor, Brom, Iod, Methyl, Ethyl, n- und i-Propyl, n-, i-, s- und t- Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy,Trichlormethoxy, Dichlorfluormethoxy, Difluorchlormethoxy,Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^5$,

wobei

$R^5$ für Methyl, Ethyl, Amino, Methylamino, Ethylamino, Dimethylamino, Diethylamino, Fluordichlormethyl, Difluormethyl, Difluorchlormethyl, Tetrafluorethyl, Trifluorchlorethyl oder Trifluormethyl steht,

n für eine Zahl 0, 1 oder 2 steht.

Het für einen gegebenenfalls ein- bis dreifach, gleich oder verschieden durch Fluor, Chlor, Brom, Nitro, Methyl, Ethyl, n- und i-Propyl, Methoxy, Ethoxy, n- oder i-Propoxy, Methylthio, Ethylthio, n- oder i- Propylthio oder Trifluormethyl substituierten Heterocyclus der Formel

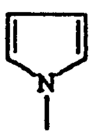 ; 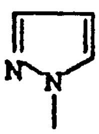 ; 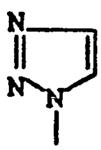 ; 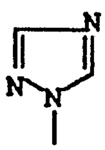 ; 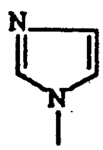 ; 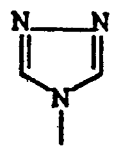 ;

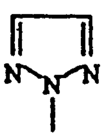 ; 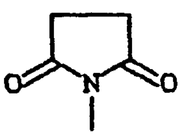 ; 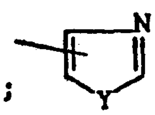 ; 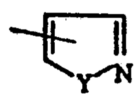 ; 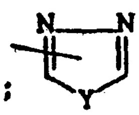 ;

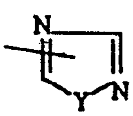 ; 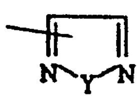 ; 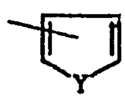 ; 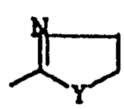 ; 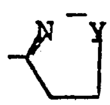 ;

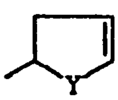 ; 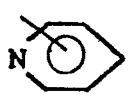 ; 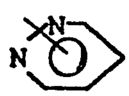 ; 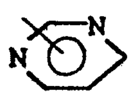 ; 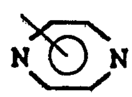 ;

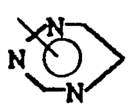 ; 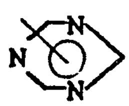 ; 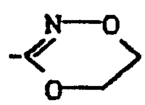 ; 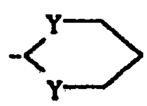 oder 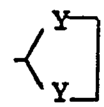

steht, wobei

Y jeweils für Sauerstoff oder Schwefel oder einen N-Alkyl-Rest mit 1 bis 4 Kohlenstoffatomen steht.

4. Verfahren zur Herstellung von 5-Amino-4-heterocyclyl-1-pyridyl-pyrazolen der Formel (I)

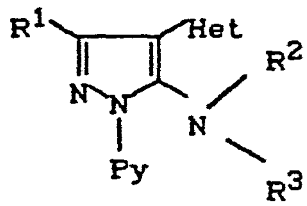

(I)

in welcher

$R^1$ für Wasserstoff oder Alkyl steht,

$R^2$ für Wasserstoff oder für einen Rest $-\overset{X}{\overset{\|}{C}}-R^4$ steht,

$R^3$ für Wasserstoff, für einen Rest $-\overset{X}{\overset{\|}{C}}-R^4$ oder für Alkyl, Alkenyl oder Alkinyl steht,

wobei

$R^4$ für Wasserstoff, Alkyl, Alkoxy, Alkylthio, Alkoxyalkyl, Alkylthioalkyl, Halogenalkyl, Alkenyl, Alkinyl, für Alkylamino, für Dialkylamino, für gegebenenfalls substituiertes Cycloalkyl, für jeweils gegebenenfalls substituiertes Aryl, Aryloxy, Arylthio, Arylamino oder Aryloxyalkyl steht und

X für Sauerstoff oder Schwefel steht,

Py für gegebenenfalls substituiertes Pyridyl steht und

Het für einen gegebenenfalls substituierten gesättigen oder ungesättigten Heterocyclus steht,

dadurch gekennzeichnet, daß man

(a) Pyridylhydrazine der Formel (II),

$$Py - NH - NH_2 \qquad (II)$$

in welcher

Py die oben angegebene Bedeutung hat,

mit Acrylnitril-Derivaten der Formel (III),

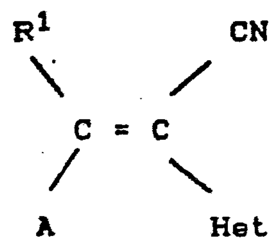

(III)

in welcher

$R^1$ und Het die oben angegebene Bedeutung haben und

A für Halogen, Hydroxy, Alkoxy oder Dialkylamino steht,

zunächst in einer 1. Stufe, gegebenenfalls in Gegenwart eines Verdünnungsmittels sowie gegebenenfalls in Gegenwart eines Reaktionshilfsmittels, umsetzt zu den Pyridylhydrazin-Derivaten der Formel (IV)

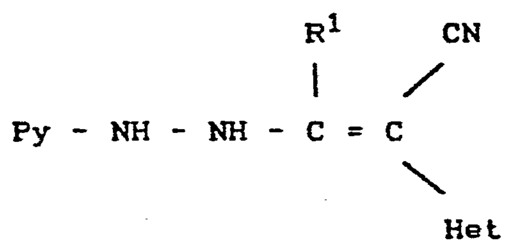

(IV)

in welcher

Py, $R^1$ und Het die oben angegebene Bedeutung haben,

und diese in einer 2. Stufe, gegebenenfalls in Gegenwart eines Verdünnungsmittels und gegebenenfalls in Gegenwart eines sauren Katalysators, cyclisiert zu den 5-Amino-4-heterocyclyl-1-pyridyl-pyrazolen der Formel (Ia)

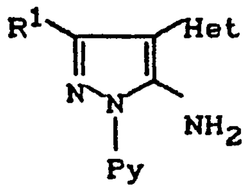

(Ia)

in welcher

$R^1$, Het und Py die oben angegebene Bedeutung haben,

oder daß man

(b) die erfindungsgemäßen 5-Amino-4-heterocyclyl-1-pyridyl-pyrazole der Formel (Ia)

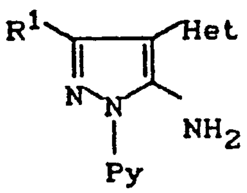

(Ia)

in welcher

$R^1$, Het und Py die oben angegebene Bedeutung haben,

mit Verbindungen der Formel (V),

$$R^{3-1} - A^1 \quad (V)$$

in welcher

$R^{3-1}$ für Alkyl, Alkenyl, Alkinyl oder einen Rest $-\overset{X}{\overset{\|}{C}}-R^4$ steht, wobei X und $R^4$ die oben angegebene Bedeutung haben und

$A^1$ für eine elektronenziehende Abgangsgruppe steht,

gegebenenfalls in Gegenwart eines Verdünnungsmittels und gegebenenfalls in Gegenwart eines Säurebindemittels, umsetzt zu den am Stickstoff alkylierten bzw. acylierten 5-Amino-4-heterocyclyl-1-pyridyl-pyrazolen der Formel (Ib),

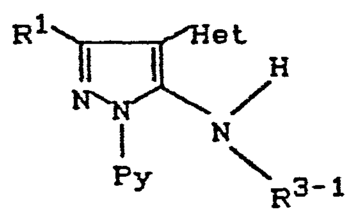

(Ib)

in welcher

$R^1$, $R^{3-1}$, Het und Py die oben angegebene Bedeutung haben,

oder daß man

(c) die erfindungsgemäßen 5-Amino-4-heterocyclyl-1-pyridyl-pyrazole der Formel (Ia)

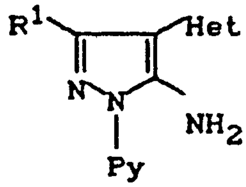

(Ia)

in welcher

$R^1$, Py und Het die oben angegebene Bedeutung haben,

mit Iso(thio)cyanaten der Formel (VI),

$$R^{4-1} - N = C = X \qquad (VI)$$

in welcher

$R^{4-1}$ für Alkyl oder für gegebenenfalls substituiertes Aryl steht und

X für Sauerstoff oder Schwefel steht,

gegebenenfalls in Gegenwart eines Verdünnungsmittels und gegebenenfalls in Gegenwart eines Reaktionshilfsmittels umsetzt zu den 5-Amino-4-heterocyclyl-1-pyridyl-pyrazolen der Formel (Ic)

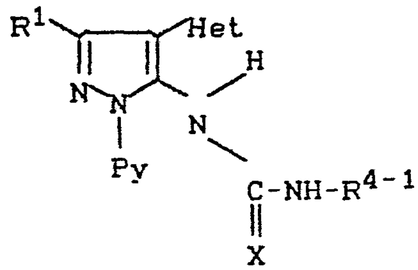

(Ic)

in welcher

$R^1$, $R^{4-1}$, Py, Het und X die oben angegebene Bedeutung haben.

5. Herbizide Mittel, gekennzeichnet durch einen Gehalt an mindestens einem 5-Amino-4-heterocyclyl-1-pyridyl-pyrazol der Formel (I) gemäß den Ansprüchen 1 bis 4.

6. Verfahren zur Bekämpfung von Unkräutern, dadurch gekennzeichnet, daß man ein 5-Amino-4-heterocyclyl-1-pyridyl-pyrazol der Formel (I) gemäß den Ansprüchen 1 bis 4 auf Unkräuter und/oder ihren Lebensraum einwirken läßt.

7. Verwendung von 5-Amino-4-heterocyclyl-1-pyridyl-pyridyl, pyrazolen der Formel (I) gemäß den Ansprüchen 1 bis 4 zur Bekämpfung von Unkräutern.

8. Verfahren zur Herstellung von herbiziden Mitteln, dadurch gekennzeichnet, daß man 5-Amino-4-heterocyclyl-1-pyridyl-pyrazole der Formel (I) gemäß den Ansprüchen 1 bis 4 mit Streckmitteln und/oder oberflächenaktiven Substanzen vermischt.